# EUROPÄISCHE PATENTANMELDUNG

(11) **EP 3 939 960 A1**
(43) Veröffentlichungstag der Anmeldung: **19.01.2022**
(21) Anmeldenummer: 20185951.9
(22) Anmeldetag: 15.07.2020
(51) Int. Cl.: C07C 209/86, C07C 263/10, C07C 265/14, C08G 69/28, C08G 59/02

(54) **VERFAHREN ZUR GEWINNUNG VON DIAMINEN AUS WÄSSRIGEN MISCHUNGEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Gewinnung mindestens eines aliphatischen, araliphatischen oder cycloaliphatischen Diamins aus einer Mischung enthaltend mindestens 35 Gew.-% Wasser und 0,2 bis 60 Gew.-% des mindestens einen aliphatischen, araliphatischen oder cycloaliphatischen Diamins durch Extraktion des mindestens einen aliphatischen, araliphatischen oder cycloaliphatischen Diamins mit mindestens einem Dicarbonsäuredinitril unter Erhalt einer wässrigen Phase enthaltend Wasser sowie eine Teilmenge des mindestens einen Dicarbonsäuredinitrils und einer organischen Phase enthaltend eine weitere Teilmenge des mindestens einen Dicarbonsäuredinitrils sowie das mindestens eine aliphatische, araliphatische oder cycloaliphatische Diamin.

## Beschreibung

Die Erfindung betrifft ein Verfahren zur Gewinnung mindestens eines aliphatischen, araliphatischen oder cycloaliphatischen Diamins aus einer wässrigen Mischung sowie die Verwendung von Dicarbonsäuredinitrilen zur Extraktion mindestens eines aliphatischen, araliphatischen oder cycloaliphatischen Diamins aus einer wässrigen Mischung und eine nach diesem Verfahren erhältliche organische Phase, enthaltend mindestens ein Dicarbonsäuredinitril, mindestens ein aliphatisches, araliphatisches oder cycloaliphatisches Diamin und Wasser sowie die Verwendung einer solchen organischen Phase als Ausgangsstoff in einer Hydrierungsreaktion.

Aliphatische, araliphatische oder cycloaliphatische Diamine sind wichtige Ausgangsmaterialen zur Herstellung von Polymeren. Dabei können sie zunächst in Diisocyanate überführt werden, welche dann zu Polyurethanen, Polyharnstoffen, Polythiourethanen oder Polyisocyanuraten umgesetzt werden. Eine weitere wichtige Anwendung von Diaminen ist die Herstellung von Polyamiden durch Polykondensation des Diamins mit Dicarbonsäuren.

Die Herstellung von Polyamiden erfolgt beispielsweise durch Aufheizen wässriger Lösungen von Diaminen und Dicarbonsäuren. Die Lösung wird dabei unter Druck erhitzt, wobei der Druck durch Entspannen von überschüssigem Wasserdampf konstant gehalten wird. Um die Polykondensation zu vervollständigen, muss das Wasser komplett aus dem Reaktionsgemisch entfernt werden. Da die Diaminkomponenten wasserdampfflüchtig sind, entsteht dabei ein Diamin-haltiges Abwasser, das aufwändig behandelt werden muss.

In CN101993178(A) ist eine solche Methode zur Abwasserbehandlung von 1,6-Hexandiaminhaltigen Abwasser beschrieben, welche eine anaerobe und eine aerobe Behandlung des Abwassers beinhaltet. Nachteilig an dem Verfahren ist, dass das enthaltene 1,6-Hexandiamin dabei zerstört wird und somit einer weiteren stofflichen Verwendung entzogen wird.

Für die Herstellung von Hexamethylendiisocyanat aus 1,6-Hexandiamin sind gewisse Anforderungen an das Diamin bekannt. So beschreibt beispielsweise die CN103922969B, dass leichtsiedende Nebenkomponenten wie Tetrahydroazepin, 1-Amino-2-cyano-1-cyclopenten oder 6-Aminocapronitril nur in sehr geringem Umfang vorhanden sein sollten. In der EP2060560B1 wird allgemein beschrieben, dass sich besonders farbhelle Isocyanate aus Aminen herstellen lassen, deren PRI-Wert, also der Gehalt an polarographisch reduzierbaren Verbindungen, weniger als 60 mol-ppm beträgt.

Andere aliphatische Diamine werden beispielsweise durch die Hydrierung oder reduktive Aminierung (auch als aminierende Hydrierung bezeichnet) entsprechender sauerstoffhaltiger Verbindungen gewonnen. Dies kann beispielsweise, wie in WO2012076315A1 beschrieben, die reduktive Aminierung von 3-Cyano-3,5,5-trimethylcyclohexanon zu 3-Aminomethyl-3,5,5-trimethylcyclohexylamin (IPDA) sein oder auch die Hydrierung von nitrogruppenhaltigen Verbindungen. Dabei entsteht neben dem gewünschten Diamin stets auch Wasser, sogenanntes Reaktionswasser. Dieses kann dann auf unterschiedliche Weise vom Amin abgetrennt werden und fällt dann als diaminhaltiger Abwasserstrom an. Sofern eine Mischungslücke besteht oder diese durch Zugabe weiterer Stoffe (beispielsweise Salze oder Lösungsmittel) herbeigeführt werden kann, bietet es sich an, zunächst eine Phasentrennung durchzuführen und später die organische Amin-Phase destillativ weiter zu trocknen. Liegt keine Mischungslücke vor, wird üblicherweise das gesamte zu entfernende Wasser destillativ entfernt, wobei in der Regel ein Teil der Diamine mit dem Destillat übergeht.

Eine weitere Quelle für wässrige Lösungen von aliphatischen, araliphatischen oder cycloaliphatischen Diaminen ist das Recycling von Polyurethanen auf Basis entsprechender Diisocyanate oder von Polyamiden, das eine immer wichtigere Rolle in der Industrie spielt. Werden diese Kunststoffe chemisch hydrolysiert, fallen wässrige Lösungen von aliphatischen, araliphatischen oder cycloaliphatischen Diaminen an, aus denen die aliphatischen, araliphatischen oder cycloaliphatischen Diamine zurückgewonnen werden müssen.

1,5-Pentandiamin wird industriell auf biotechnologischem Weg hergestellt und fällt als wässrige Lösung an. In EP2684867A1 wird beispielsweise ein Verfahren zur Herstellung von 1,5-Pentandiamin beschrieben, bei dem das Diamin in einer Flüssigphasenextraktion aus der wässrigen Phase extrahiert wird. Als Extraktionsmittel werden halogenfreie aliphatische Lösungsmittel, vorzugsweise unverzweigte Alkohole mit 4 bis 7 Kohlenstoffatomen, empfohlen. Diese Extraktionsmittel müssen anschließend aufwändig vom Produkt abgetrennt werden.

Auch die EP3235804A1 beschriebt eine Extraktion von 1,5-Pentandiamin aus wässriger Phase. Im Anschluss an die Extraktion wird hier jedoch eine Reduktion durchgeführt, um 2,3,4,5-Tetrahydropyridin abzubauen, die für spätere Verfärbungen der ausgehend von dem 1,5-Pentandiamin hergestellten Polyamide verantwortlich gemacht werden. Aus diesem Grund wird an das Extraktionsmittel die Anforderung gestellt, sich in der chemischen Hydrierung inert zu verhalten. Erneut werden Alkohole wie beispielsweise Ethanol oder Butanol als Extraktionsmittel bevorzugt.

Ausgehend von diesem Stand der Technik lag der vorliegenden Erfindung die Aufgabe zu Grunde, ein wirtschaftlich und ökologisch sinnvolleres Verfahren zur Gewinnung von aliphatischen, araliphatischen oder cycloaliphatischen Diaminen aus einer wässrigen Mischung bereitzustellen.

Diese Aufgabe wurde gelöst durch ein Verfahren zur Gewinnung mindestens eines aliphatischen, araliphatischen oder cycloaliphatischen Diamins aus einer Mischung enthaltend mindestens 35 Gew.-% Wasser und 0,2 bis 60 Gew.-% des mindestens einen aliphatischen, araliphatischen oder cycloaliphatischen Diamins durch Extraktion des mindestens einen aliphatischen, araliphatischen oder cycloaliphatischen Diamins mit mindestens einem Dicarbonsäuredinitril unter Erhalt einer wässrigen Phase enthaltend Wasser sowie eine Teilmenge des mindestens einen Dicarbonsäuredinitrils und einer organischen Phase enthaltend eine weitere Teilmenge, bevorzugt die verbleibende Teilmenge des mindestens einen Dicarbonsäuredinitrils sowie das mindestens eine aliphatische, araliphatische oder cycloaliphatische Diamin.

Bevorzugt bedeuten erfindungsgemäß die Ausdrücke "umfassend" oder "enthaltend", "im Wesentlichen bestehend aus" und besonders bevorzugt "bestehend aus".

*Gehaltsangaben organischer Verbindungen* beziehen sich im Rahmen der vorliegenden Erfindung, sofern nicht anders angegeben, auf per Gaschromatographie bestimmte Werte. Die quantitative Auswertung von Gaschromatogrammen, gegebenenfalls mit Hilfe eines internen Standards, ist dem Fachmann bekannt. Gegebenenfalls erforderliche Methoden zur Bestimmung des *Wassergehalts* sind dem Fachmann ebenfalls bekannt. Die Methoden des Standes der Technik können auch im Rahmen der vorliegenden Erfindung angewandt werden. Im Zweifelsfall ist der per Karl-Fischer-Titration bestimmte Wassergehalt maßgeblich. Die Anwesenheit von Aminen kann zu schleppenden Endpunkten führen. In solchen Fällen ist der per Karl-Fischer-Titration nach Pufferung mit wasserfreier Benzoesäure bestimmte Wert maßgeblich. Das Verfahren unter Pufferung mit Benzoesäure ist dem Fachmann ebenfalls bekannt. Zur Karl-Fischer-Titration allgemein, siehe Jander, Jahr, Maßanalyse, 17. Aufl., de Gruyter, Berlin (2009), S. 279 bis S. 282).

"Mindestens ein", wie hierin verwendet, bezieht sich auf 1 oder mehr, beispielsweise 2, 3, 4, 5, 6, 7, 8, 9 oder mehr. Im Zusammenhang mit Bestandteilen der hierin beschriebenen Verbindungen bezieht sich diese Angabe nicht auf die absolute Menge an Molekülen, sondern auf die Art des Bestandteils. "Mindestens ein aliphatisches, araliphatisches oder cycloaliphatisches Diamin" bedeutet daher beispielsweise, dass nur eine Art von Diamin oder mehrere verschiedene Arten von Diaminen, ohne Angaben über die Menge der einzelnen Verbindungen zu machen, enthalten sein können.

Zahlenwerte, die hierin ohne Dezimalstellen angegeben sind, beziehen sich jeweils auf den vollen angegebenen Wert mit einer Dezimalstelle. So steht beispielsweise "99%" für "99,0%".

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Vorzugsweise ist das mindestens eine Dicarbonsäuredinitril ein Dicarbonsäuredinitril gemäß der Formel (I),

NC-R-CN (I),

wobei R für einen Alkylrest mit 1 bis 12 Kohlenstoffatomen, einen Alkenylrest mit 4 bis 8 Kohlenstoffatomen oder einen Arylrest mit 6 Kohlenstoffatomen steht. Auch Mischungen solcher Dicarbonsäuredinitrile können erfindungsgemäß eingesetzt werden.

In einer bevorzugten Ausführungsform der Erfindung setzt man Dicarbonsäuredinitrile gemäß der Formel (I) ein, wobei R für einen geraden oder verzweigten Alkylrest mit 2 bis 6 Kohlenstoffatomen, einen Alkenylrest mit 4 Kohlenstoffatomen oder einen Arylrest mit 6 Kohlenstoffatomen steht.

In einer besonders bevorzugten Ausführungsform ist das mindestens eine Dicarbonsäuredinitril ausgewählt aus der Gruppe bestehend aus Succinonitril, Glutaronitril, Adiponitril, Sebaconitril, Methylglutaronitril, Ethylsuccinonitril und Methylenglutaronitril.

In einer ganz besonders bevorzugten Ausführungsform ist das mindestens eine Dicarbonsäuredinitril ausgewählt aus der Gruppe bestehend aus Glutaronitril, Methylenglutaronitril und Adiponitril.

Die Herstellung solcher Dicarbonsäuredinitrile ist dem Fachmann bekannt. Industriell erfolgt sie beispielsweise durch Addition von Cyanwasserstoff an geeignete Ausgangsstoffe, wie beispielsweise Acrylnitril oder Butadien, oder durch katalytische Oxidation von Alkenen und Ammoniak mit Luft. Auch Umsetzungen von Chlorcyan, beispielsweise mit Acetonitril zum Malonitril, sind als Wege zur Herstellung von Dicarbonsäuredinitrilen beschrieben oder Substitutionsreaktionen von Halogenalkanen mit Alkalicyaniden.

Glutaronitril für die Extraktion des mindestens einen aliphatischen, araliphatischen oder cycloaliphatischen Diamins mit mindestens einem Dicarbonsäuredinitril kann im Grunde auf beliebige, dem Fachmann bekannte Weise hergestellt werden. Es kann beispielsweise durch Umsetzung einer Lösung von 1,3-Dichlorpropan mit Kaliumcyanid hergestellt werden. Zur Isolierung kann dann das Glutaronitril beispielsweise zunächst in einer Flüssig-Flüssig-Extraktion mit Wasser und Methylenchlorid extrahiert werden. Dabei kann bei Bedarf das Wasser mit weiteren Salzen, beispielsweise Natriumchlorid oder Kaliumchlorid, versetzt werden, um die Phasentrennung zu verbessern. Nach Trennung der Phasen kann die glutaronitrilhaltige organische Phase, gegebenenfalls nach Trocknung, destillativ aufgearbeitet werden, um das Glutaronitril in reiner Form zu erhalten.

Methylenglutaronitril für die Extraktion des mindestens einen aliphatischen, araliphatischen oder cycloaliphatischen Diamins mit mindestens einem Dicarbonsäuredinitril kann im Grunde auf beliebige, dem Fachmann bekannte Weise hergestellt werden. Es kann beispielsweise in einer katalytischen Dimerisierung von Acrylnitril in Gegenwart von Tricyclohexylphosphin oder von Metallhalogeniden und Trialkylaminen gewonnen werden. Die Aufarbeitung des Methylenglutaronitril erfolgt beispielsweise, wie in Spalte 3, Zeile 35 bis Spalte 4, Zeile 41 der US4422981 beschrieben, extraktiv in Gegenwart von Wasser. Die organische Phase kann später zur Reindarstellung des Methylenglutaronitrils zum Beispiel einer Vakuumdestillation unterzogen werden. In einem solchen Verfahren lässt sich auch die Behandlung des bei der Extraktion des mindestens einen aliphatischen, araliphatischen oder cycloaliphatischen Diamins mit mindestens einem Dicarbonsäuredinitril anfallenden, Methylenglutaronitril enthaltenden Abwasserstroms gut in den Prozess integrieren.

Adiponitril für die Extraktion des mindestens einen aliphatischen, araliphatischen oder cycloaliphatischen Diamins mit mindestens einem Dicarbonsäuredinitril kann im Grunde auf beliebige, dem Fachmann bekannte Weise hergestellt werden. Industriell etabliert haben sich einerseits die katalytische Hydrocyanierung von 1,3-Butadien und die Dimerisierung von Acrylnitril. Bei der Dimerisierung von Acrylnitril wird zwischen der katalytischen Dimerisierung, in welcher zunächst Hexendinitril entsteht, das anschließend hydriert wird, und der elektrochemischen Hydrodimerisierung, welche direkt zum Adiponitril führt, unterschieden. Bevorzugt wird für die erfindungsgemäße Extraktion des mindestens einen aliphatischen, araliphatischen oder cycloaliphatischen Diamins ein Adiponitril verwendet, welches auf dem Wege der elektrochemischen Hydrodimerisierung hergestellt wurde. In diesem Verfahren lässt sich auch die Behandlung des bei der Extraktion des mindestens einen aliphatischen, araliphatischen oder cycloaliphatischen Diamins mit Adiponitril anfallenden, Adiponitril enthaltenden Abwasserstroms gut in den Prozess integrieren.

Mischungen enthaltend mindestens 35 Gew.-% Wasser und 0,2 bis 60 Gew.-% mindestens eines aliphatischen, araliphatischen oder cycloaliphatischen Diamins fallen, wie zuvor beschrieben, in ganz verschiedenen chemischen Prozessen, beispielsweise als Abwasserstrom oder als Rohproduktstrom, an. Für das erfindungsgemäße Verfahren eignen sich insbesondere solche Mischungen die einen Gehalt des mindestens einen aliphatischen, araliphatischen oder cycloaliphatischen Diamins von ≤ 40 Gew.-%, bevorzugt ≤ 25 Gew.-%, besonders bevorzugt ≤ 12 Gew.-%, bezogen auf die Gesamtmasse der Mischung, aufweisen. Gleichzeitig ist der Gehalt vorzugsweise ≥ 0,5 Gew.-%, bevorzugt ≥ 1 Gew.-% und besonders bevorzugt ≥ 2 Gew.-%, bezogen auf die Gesamtmasse der Mischung. Bei höheren Gehalten an aliphatischen, araliphatischen oder cycloaliphatischen Diaminen gewinnt aus wirtschaftlichen Gründen die destillative Aufbereitung der Mischung zur Abtrennung der aliphatischen, araliphatischen oder cycloaliphatischen Diamine an Bedeutung, während bei geringeren Gehalten die Abtrennung des mindestens einen Diamins aus der Mischung an sich ihre Wirtschaftlichkeit verliert. Neben dem mindestens einen aliphatischen, araliphatischen oder cycloaliphatischen Diamin und Wasser enthält die wässrige Phase in der Regel noch geringe Mengen anderer Verbindungen. Dies können beispielsweise Alkohole, Glycole, Aminoalkohole, Imine, Kohlenhydrate, Nitroverbindungen, anorganische oder organische Säuren, Carbonsäureester, Carbonsäureamide, anorganische oder organische Basen, insbesondere primäre oder sekundäre Amine, polarographisch reduzierbare Verbindungen wie beispielsweise 2,3,4,5-Tetrahydropyridin, Tetrahydroazepin, 1-Amino-2-cyano-1-cyclopenten oder 6-Aminocapronitril, Urethane oder Harnstoffe sein.

Das aliphatische, cycloaliphatische oder araliphatische Diamin ist vorzugsweise ausgewählt aus der Gruppe bestehend aus 1,4-Butandiamin, Neopentandiamin, 1,5-Pentandiamin (PDA), 1,5-Diamino-2-methylpentan, 1,5-Diamino-2-butyl-2-ethylpentan, 1,6-Hexandiamin (HDA), 2,5-Diamino-2,5-dimethylhexan, 1-Amino-3,3,5-trimethyl-5-aminomethyl-cyclohexan (IPDA), 1,4-Diaminocyclohexan, 2,4-Diamino-1-methylcyclohexan, 2,6-Diamino-1-methylcyclohexan, m-Hexahydroxylylendiamin, 1,6-Diamino-2,2,4-trimethylhexan, 1,6-Diamino-2,4,4-trimethylhexan, 2,4'-Diaminodicyclohexylmethan, 4,4'-Diaminodicyclohexylmethan, Bis(aminomethyl)bicyclo[2.2.1]heptan (NBDA), 1,8-Diaminooctan, 1,9-Diaminononan, 1,10-Diaminodecan, 1,11-Diaminoundecan, 1,12-Diaminododecan und m-Xylylendiamin (XDA).

Bevorzugt ist das aliphatische, cycloaliphatische oder araliphatische Diamin ein aliphatisches Diamin ausgewählt aus der Gruppe bestehend aus 1,4-Butandiamin, 1,5-Pentandiamin, 1,5-Diamino-2-methylpentan und 1,6-Hexandiamin. Besonders bevorzugt ist das aliphatische Diamin 1,5-Pentandiamin oder 1,6-Hexandiamin.

Die Mischung enthaltend mindestens 35 Gew.-% Wasser und 0,2 bis 60 Gew.-% des mindestens einen aliphatischen, araliphatischen oder cycloaliphatischen Diamins wird erfindungsgemäß einer Extraktion zugeführt, in der das in der Mischung enthaltene mindestens eine aliphatische, araliphatische oder cycloaliphatische Diamin mit mindestens einem Dicarbonsäuredinitril in Kontakt gebracht und so extrahiert wird. Bei der Extraktion werden eine wässrige Phase enthaltend Wasser und eine Teilmenge des mindestens einen Dicarbonsäuredinitrils sowie eine organische Phase enthaltend eine weitere Teilmenge, bevorzugt die verbleibende Teilmenge, des mindestens einen Dicarbonsäuredinitrils, das mindestens eine aliphatische, araliphatische oder cycloaliphatische Diamin und Wasser gebildet und voneinander getrennt. Bevorzugt befinden sich in der getrennten organischen Phase 75 bis 100%, besonders bevorzugt 95 bis 100% und ganz besonders bevorzugt 98 bis 99,99% des ursprünglich in der wässrigen Mischung enthaltenen, mindestens einen aliphatischen, araliphatischen oder cycloaliphatischen Diamins.

Die organische Phase, also das Extrakt, enthaltend die eine weitere Teilmenge, bevorzugt die verbleibende Teilmenge, des mindestens einen Dicarbonsäuredinitrils, das mindestens eine aliphatische, araliphatische oder cycloaliphatische Diamin und Wasser ist ebenfalls Gegenstand der Erfindung. Der Wassergehalt des Extrakts ergibt sich im Wesentlichen aus der Löslichkeit von Wasser in dem mindestens einen Dicarbonsäuredinitril unter den Extraktionsbedingungen. Bevorzugt beträgt der Wassergehalt des Extrakts ≤ 25 Gew.-%, besonders bevorzugt ≤ 12 Gew.-%, ganz besonders bevorzugt ≤ 8 Gew.-% und am meisten bevorzugt ≤ 6 Gew.-% Wasser bezogen auf die Gesamtmasse des Extrakts. In der Regel liegt der Wassergehalt des Extrakts nicht unter 0,2 Gew.-%, bevorzugt nicht unter 0,5 Gew.-%, besonders bevorzugt nicht unter 1 Gew.-% und ganz besonders bevorzugt nicht unter 2 Gew.-% Wasser bezogen auf die Gesamtmasse des Extrakts.

Die Extraktion erfolgt vorzugsweise als Flüssig-Flüssig-Extraktion, bei der die Mischung enthaltend das mindestens eine aliphatische, araliphatische oder cycloaliphatische Diamin mit dem mindestens einem Dicarbonsäuredinitril in Kontakt gebracht wird. Dies kann als Batch, semikontinuierlich oder kontinuierlich erfolgen, wobei die beiden Phasen gemischt werden.

Für die Extraktion können alle dem Fachmann bekannten Methoden und Apparaturen, wie z.B. Mixer-Settler oder Extraktionskolonnen eingesetzt werden. Die Extraktion kann einstufig oder mehrstufig im Gleich- oder Gegenstrom erfolgen. Bevorzugt erfolgt die Extraktion im Gegenstrom, besonders bevorzugt ist eine mehrstufige Extraktion im Gegenstrom.

Das Mischen der beiden Phasen kann auf beliebige Art und Weise erfolgen, es können beispielsweise Statikmischer, Mischdüsen, Rührer oder Mischpumpen verwendet werden. Zur Absenkung der notwendigen Trenn- und Verweilzeiten können die verwendeten Trennapparate (Abscheider) mit Koaleszenzhilfen wie beispielsweise Gestricken, Platten oder Füllkörpern versehen werden. Es ist ebenfalls möglich, die Phasentrennung in einer Zentrifuge herbeizuführen oder durch Anlegen eines elektrischen Feldes zu verbessern.

Geeignete Vorrichtungen für die Extraktion sind dem Fachmann bekannt. Bevorzugt werden Mixer/Settler oder Extraktionskolonnen eingesetzt. Die Extraktion wird im Allgemeinen bei Temperaturen von 10 bis 110 °C, bevorzugt 20 bis 80 °C und besonders bevorzugt 22 bis 45 °C durchgeführt. Sofern zur Extraktion ein bei Raumtemperatur festes Dicarbonsäuredinitril wie beispielsweise Succinonitril verwendet wird, ist es vorteilhaft, die Extraktion bei einer erhöhten Temperatur, vorzugsweise im Bereich von 60 bis 80 °C, durchzuführen, um eine Kristallisation des bei Raumtemperatur festen Dicarbonsäuredinitrils zu vermeiden. In vielen Fällen, aber insbesondere bei der Verwendung von Succinonitril als Extraktionsmittel, ist die Anwesenheit eines Salzes zur Verbesserung der Phasentrennung vorteilhaft. Der Druck beträgt im Allgemeinen 1 bis 6 bar(a), bevorzugt 1 bis 3 bar(a) und besonders bevorzugt 1 bis 1,2 bar(a). Ganz besonders bevorzugt erfolgt die Extraktion also bei einer Temperatur von 25 bis 45 °C und einem Druck von 1 bis 1,2 bar(a).

In einer weiteren bevorzugten Ausführungsform der Erfindung wird die Mischung enthaltend das mindestens eine aliphatische, araliphatische oder cycloaliphatische Diamin 1 bis 30 Mal, bevorzugt 1 bis 10 Mal und besonders bevorzugt 2 bis 8 Mal mit dem mindestens einen Dicarbonsäuredinitril extrahiert bzw. in einer Extraktionskolonne mit entsprechender Anzahl an Extraktionsstufen extrahiert. Das Volumenverhältnis der Mischung enthaltend das mindestens eine aliphatische, araliphatische oder cycloaliphatische Diamin zu dem mindestens einen Dicarbonsäuredinitril liegt im Bereich von 6:1 bis 1:12, bevorzugt im Bereich von 2:1 bis 1:6 und besonders bevorzugt im Bereich von 1:1 bis 1:3. Um die Volumenverhältnisse einzustellen, ist es auch möglich, einen Teil der in der Extraktion erhaltenen wässrigen und/oder der organischen Phase im Kreis zu fahren. Die Rückführung kann dabei in die gleiche Extraktionsstufe oder in eine andere, weiter stromaufwärts gelegene Extraktionsstufe erfolgen.

Bevorzugt erfolgt die Extraktion im alkalischen Milieu, das heißt bei einem pH-Wert > 7, bevorzugt bei einem pH-Wert ≥ 9, besonders bevorzugt bei einem pH-Wert ≥ 10 und ganz besonders bevorzugt bei einem pH-Wert ≥ 11. Vorzugsweise erfolgt die Einstellung des alkalischen Milieus durch Zugabe einer anorganischen Base, vorzugsweise durch Zugabe eines oder mehrerer Alkalimetallhydroxide, besonders bevorzugt durch Zugabe von Natriumhydroxid. Die Zugabe der Base kann in reiner Form oder als Lösung, bevorzugt als wässrige Lösung erfolgen.

Auf diese Weise werden mit Hilfe des erfindungsgemäßen Verfahrens 75 bis 100%, bevorzugt 95 bis 100% und besonders bevorzugt 98 bis 99,99% des ursprünglich in der wässrigen Mischung enthaltenen, mindestens einen aliphatischen, araliphatischen oder cycloaliphatischen Diamins aus der wässrigen Phase extrahiert.

Die nach der Extraktion erhaltene wässrige Phase enthält kein oder nur noch geringe Mengen des mindestens einen aliphatischen, araliphatischen oder cycloaliphatischen Diamins und eine geringe Menge des mindestens einen Dicarbonsäuredinitrils. Der Gehalt an Dicarbonsäuredinitril hängt unter anderem vom Dicarbonsäuredinitril und dessen Löslichkeit in Wasser sowie von den gewählten Verfahrensbedingungen, insbesondere der Temperatur, ab. Er liegt beispielsweise zwischen 0,1 und 20 Gew.-%, bevorzugt zwischen 0,2 und 10 Gew.-% und besonders bevorzugt zwischen 1 und 5 Gew.-%. Der Gehalt an aliphatischem, araliphatischem oder cycloaliphatischem Diamin in der wässrigen Phase hängt unter anderem vom dem Ursprungsgehalt an aliphatischem, araliphatischen oder cycloaliphatischen Diamin in der zu extrahierenden Mischung ab und kann bei hohen Ursprungsgehalten beispielsweise bis zu 5,00 Gew.-% betragen. Bevorzugt beträgt der Gehalt an aliphatischem, araliphatischem oder cycloaliphatischem Diamin in der wässrigen Phase zwischen 0,0005 und 0,5 Gew.-%, besonders bevorzugt zwischen 0,001 und 0,2 Gew.-% und ganz besonders bevorzugt zwischen 0,001 und 0,1 Gew.-% bezogen auf die Gesamtmasse der wässrigen Phase, wobei das Amin als freies Amin oder in Form eines sauren Salzes vorliegen kann. Diese wässrige Phase kann nun vorzugsweise an anderer Stelle beispielsweise für eine Extraktion eingesetzt werden. Alternativ kann sie beispielsweise destillativ vorgereinigt und dann einer biologischen Abwasserbehandlung zugeführt werden.

In einer bevorzugten Ausführungsform der Erfindung wird die wässrige Phase, gegebenenfalls nach Durchführung weiterer Zwischenschritte, wie beispielsweise einer Neutralisation, Filtration oder Entsalzung, in einem Verfahren zur Herstellung des dem in der wässrigen Phase enthaltenen Dicarbonsäuredinitril entsprechenden Dicarbonsäuredinitrils in einem Extraktionsschritt eingesetzt. Beispielsweise wird also eine wässrige Phase enthaltend Glutaronitril vorzugsweise in einer Flüssig-Flüssig-Extraktion von Glutaronitril in einem Verfahren zur Herstellung von Glutaronitril verwendet, eine wässrige Phase enthaltend Metyhlenglutaronitril vorzugsweise in einer Flüssig-Flüssig-Extraktion in einem Verfahren zur Herstellung von Methylenglutaronitril, eine wässrige Phase enthaltend Adiponitril vorzugsweise in einer Flüssig-Flüssig-Extraktion in einem Verfahren zur Herstellung von Adiponitril, und so weiter.

Wie eingangs beschrieben, kann beispielsweise die Reinigung von Glutaronitril eine Flüssig-Flüssig-Extraktion in Gegenwart von Wasser und einem organischen Lösungsmittel wie beispielsweise Methylenchlorid umfassen. In einer weiteren besonders bevorzugten Ausführungsform der Erfindung, bei der Glutaronitril als Dicarbonsäuredinitril eingesetzt wird, wird die in der Extraktion anfallende wässrige Phase enthaltend Glutaronitril in der Flüssig-Flüssig-Extraktion im Rahmen eines Herstellverfahrens von Glutaronitril verwendet, indem sie zumindest einen Teil des ansonsten in der Flüssig-Flüssig-Extraktion verwendeten Wassers ersetzt. Das in der wässrigen Phase enthaltene Glutaronitril wird dabei zusammen mit dem Reaktionsprodukt aus der Herstellung von Glutaronitril in die organische Phase extrahiert und kann ohne weiteres zusammen mit diesem weiter aufgearbeitet werden. Das in der wässrigen Phase enthaltene Glutaronitril wird so auf ökonomische Weise einer stofflichen Verwendung zugeführt.

In einer ebenfalls bevorzugten Ausführungsform der Erfindung wird die wässrige Phase enthaltend Dicarbonsäuredinitril in einem Verfahren zur Herstellung von Adiponitril in einem Extraktionsschritt eingesetzt. So ist es beispielsweise üblich, den Ablauf aus der Kathodenzelle einer Elektrohydrodimerisation in Gegenwart von Acrylnitril mit Wasser zu extrahieren, um quartäre Ammoniumsalze aus dem Produktstrom zu entfernen und in den Prozess zurückzuführen (vgl. Albright's Chemical Engineering Handbook, CRC Press, ISBN: 978-0-8247-5362-7, Seite 1784). Beispielhaft sei an dieser Stelle auf ein Verfahren zur Herstellung von Adiponitril hingewiesen, wie es in US3267131 beschrieben ist. Dort sind verschiedene Varianten eines solchen Verfahrens ausführlich beschrieben. In einer ersten Variante wird zum Beispiel der Ablauf aus einer Zelle zur Elektrohydrodimerisierung von Acrylnitril mit Acrylnitril und Wasser verdünnt und dann die sich bildenden Acrylnitril-Phase im Gegenstrom mit Wasser extrahiert, um quartäres Ammoniumsalz, das sich in der wässrigen Phase anreichert, vom Reaktionsprodukt, das sich in der Acrylnitril-Phase anreichert, zu trennen (vgl. US3267131, Spalte 8, Zeilen 44-56). In einer weiteren Variante, wird der Ablauf aus der Kathodenzelle zunächst mit Acrylnitril extrahiert, um einen wässrigen Strom zu erzeugen, der im Wesentlichen frei von Adiponitril ist und nur noch das quartäre Ammoniumsalz enthält. Der Acrylnitril-Strom wird dann wiederum mit Wasser extrahiert, um einen Produktstrom aus Acrylnitril und Adiponitril zu erzeugen, der im Wesentlichen frei von quartärem Ammoniumsalz ist (vgl. US3267131, Spalte 9, Zeilen 38-52). Die in der Extraktion der vorliegenden Erfindung anfallende wässrige Phase enthaltend eine Teilmenge des mindestens einen Dicarbonsäuredinitrils eignet sich hervorragend, für den Einsatz in einer der zuvor beschriebenen Extraktionen von quartären Ammoniumsalzen aus der Kathodenflüssigkeit einer Elektrohydrodimerisierung. In der wässrigen Phase enthaltenes Dicarbonsäuredinitril geht dabei in das Rohprodukt über und stört die Elektrohydrodimerisierung deshalb nicht.

Sofern es sich bei dem Dicarbonsäuredinitril nicht um Adiponitril handelt, ergeben sich verschiedene Möglichkeiten zum weiteren Vorgehen. Das Dicarbonsäuredinitril kann beispielsweise destillativ aus dem Rohprodukt abgetrennt werden. Da es ohnehin üblich ist, das Rohprodukt zu destillieren, ist der zusätzliche Aufwand hierfür gering. Alternativ ist es auch möglich, das Dicarbonsäuredinitril zumindest teilweise im Adiponitril zu belassen und gemeinsam mit diesem einer Hydrierung zu unterziehen, wobei dann ein Diamin aus dem Dicarbonsäuredinitril und 1,6-Hexandiamin aus dem Adiponitril entstehen. An dieser Stelle ergibt sich die Möglichkeit, Das Diamin destillativ vom 1,6-Hexandiamin abzutrennen und einer weiteren Verwendung zuzuführen. Ebenso ist es denkbar, die Mischung der beiden Diamine, ohne sie vollständig voneinander zu trennen, für die Herstellung von Copolymeren, also Polymeren mit unterschiedlichen Monomerbausteinen, in diesem Falle also zwei oder mehr unterschiedlichen Diaminen, einzusetzen.

Beispielhaft sei hier eine Ausführungsform erläutert, in der Glutaronitril als Dicarbonsäuredinitril zur Extraktion von 1,5-Pentandiamin aus einer wässrigen Phase eingesetzt wird. In der Extraktion wird dann eine wässrige Phase enthaltend Glutaronitril erhalten, die dann wiederum im Rahmen eines Herstellverfahrens von Adiponitril für eine Flüssig-Flüssig-Extraktion verwendet wird. Auf diese Weise können also wie zuvor beschrieben, quartäre Ammoniumsalze aus der Kathodenflüssigkeit abgetrennt werden und man erhält in dieser Flüssig-Flüssig-Extraktion einen Rohproduktstrom enthaltend Adiponitril (aus dem Herstellverfahren für Adiponitril) und Glutaronitril (aus der wässrigen Phase enthaltend Glutaronitril). In der destillativen Aufarbeitung dieses Rohproduktstroms können Glutaronitril und Adiponitril getrennt werden. Erfolgt an dieser Stelle keine Auftrennung der beiden verschiedenen Nitrile, so können diese gemeinsam einer Hydrierung zu den entsprechenden Diaminen 1,5-Pentandiamin und 1,6-Hexandiamin unterzogen werden und dann entweder destillativ voneinander getrennt oder gemeinsam weiter zu Copolymeren umgesetzt werden.

Besonders bevorzugt ist die Ausführungsform der Erfindung, bei der als Dicarbonsäuredinitril Adiponitril eingesetzt wird und dann die wässrige Phase enthaltend Adiponitril in einem Verfahren zur Herstellung von Adiponitril wie zuvor beschrieben genutzt wird. In dieser bevorzugten Ausführungsform, geht das Adiponitril aus der Extraktion des Diamins in das Rohprodukt der Adiponitril-Herstellung über und wird so direkt einer stofflichen Verwertung zugeführt. Die Aufarbeitung kann dann ohne weiteres zusammen mit dem Adiponitril aus dem Verfahren zur Herstellung von Adiponitril erfolgen und eine spätere Hydrierung des Adiponitrils liefert 1,6-Hexandiamin als alleiniges Hauptprodukt.

Die nach der Extraktion des aliphatischen Diamins im erfindungsgemäßen Verfahren erhaltene organische Phase enthält neben der weiteren, bevorzugt der verbleibenden Teilmenge des mindestens einen Dicarbonsäuredinitrils nun das extrahierte mindestens eine aliphatische, araliphatische oder cycloaliphatische Diamin und Wasser.

Ein weiterer Gegenstand der Erfindung ist eine organische Phase, bestehend zu 1 bis 50 Gew.-% aus mindestens einem aliphatischen, araliphatischen oder cycloaliphatischen Diamin, zu 0,2 bis 25 Gew.-% aus Wasser und zu 0,01 bis 5 Gew.-% aus Verunreinigungen, wobei der zu 100 Gew.-% fehlende Teil der organischen Phase aus mindestens einem Dicarbonsäuredinitril besteht.

Als Verunreinigungen können beispielsweise Salze, Alkali- oder Erdalkalimetallhydroxide, Kohlenhydrate, Imine, Alkohole, Nitroverbindungen, Glycole, Aminoalkohole, Carbonsäureester, Ether, Carbonsäureamide, Monoamine, sekundäre Amine oder chlorierte Kohlenwasserstoffe auftreten.

In einer bevorzugten Ausführungsform der Erfindung wird diese organische Phase, gegebenenfalls nach Durchlaufen eines oder mehrerer Zwischenschritte, einer Hydrierungsreaktion unter Erhalt eines Hydrierungsproduktes, enthaltend das mindestens eine aliphatische, araliphatische oder cycloaliphatische Diamin, unterzogen. Optionale Zwischenschritte sind beispielsweise Abmischung mit weiterem Dicarbonsäuredinitril, Reinigungsschritte, Trocknungsschritte oder Zwischenlagerungen. Für diese Hydrierung zu den entsprechenden Diaminen sind verschiedene Verfahren bekannt. Handelt es sich bei dem mindestens einen Dicarbonsäuredinitril um ein alkenylsubstituiertes Dicarbonsäuredinitril, so kann zunächst die Hydrierung der Doppelbindung der Alkenylgruppe zur Alkylgruppe und anschließend die Hydrierung der Nitrilgruppen zu den Aminen erfolgen. Während die Hydrierung der Doppelbindung unter relativ milden Bedingungen beispielsweise an Pd/C erfolgen kann, erfordert die Hydrierung der Nitrilgruppen harschere Bedingungen und die Anwesenheit von Aminen oder Ammoniak. Alternativ können die Doppelbindung und das Nitril auch in einem Schritt zum entsprechenden Alkyldiamin hydriert werden.

Handelt es sich bei dem mindestens einen aliphatischen, araliphatischen oder cycloaliphatischen Diamin um m-Xylylendiamin, so ist es möglich, die Hydrierbedingungen so zu wählen, dass dieses Xylylendiamin gleichzeitig zum 1,3-Bis(aminomethyl)cyclohexan hydriert wird, welches sich beispielsweise durch Phosgenierung zum 1,3-Bis(isocyanatomethyl)cyclohexan (H₆XDI) umsetzen lässt.

Aus US3696153 A ist die Hydrierung von Adiponitril bei Temperaturen von 100 bis 200 °C und einem Druck von ca. 340 atm (vgl. Spalte 3, Zeilen 20-32) in Gegenwart von Ammoniak an einem eisenhaltigen Katalysator (vgl. Spalte 2, Zeilen 19-27) bekannt. Ähnliche Verfahren finden sich auch in US4064172 (vgl. Spalte 2, Zeilen 20-30).

In US3255248 ist ebenfalls ein katalytisches Hydrierverfahren beschrieben, mit dem sich vorzugsweise Kohlenwasserstoffbasierte Nitrile mit 2-20 Kohlenstoffatomen und 1 oder 2 Nitrilgruppen (vgl. Spalte 2, Zeilen 11-14) zu den entsprechenden Aminen hydrieren lassen. Das Verfahren wird bei 40 - 250 °C und einem Druck oberhalb von 20 atm ausgeführt (vgl. Spalte 2, Zeilen 40-69). Als Katalysatoren werden gesinterte Metallkatalysatoren auf Basis von Nickel und/oder Kobalt verwendet (vgl. Spalte 3, Zeilen 1-21).

In US3232888 ist die Verwendung Kobalt-basierter Katalysatoren für die Hydrierung von Nitrilen oder Dinitrilen in flüssigem Ammoniak beschrieben. In Beispiel 1 wird gezeigt, dass die Hydrierung auch in Gegenwart größerer Mengen des Produkts der Hydrierung, in dem Fall 1,6-Hexandiamin funktioniert. In Beispiel 2 ist die Hydrierung von 1,8-Octandinitril beschrieben.

Ein weiteres wichtiges Verfahren zur Hydrierung von Dicarbonsäuredinitrilen ist beispielsweise in DE69413560T2 beschrieben und nutzt dotierte, auf Raney-Nickel basierte Katalysatoren (vgl. letzte drei Absätze auf Seite 7). Die Hydrierung verläuft in Gegenwart eines Alkalimetallhydroxids (vgl. Seite 8, Absatz 3), bis zu 50% Wasser (vgl. Seite 8, Absatz 6) sowie des angestrebten Amins (vgl. Seite 9, Absatz 3) bei relativ milden Bedingungen, d.h. bei einer Temperatur unterhalb von 150 °C, vorzugsweise unterhalb von 100 °C (vgl. Seite 9, letzte 2 Absätze) und einem Wasserstoffdruck von 1 bis 100 bar (vgl. Seite 10, Absatz 1). Beispielhaft sind 80 °C und 2,5 MPa Wasserstoff offenbart (Seite 11f, Beispiel 2).

In einer bevorzugten Ausführungsform des erfindungsgemäßen Verfahrens wird die organische Phase aus der Extraktion, die eine weitere Teilmenge, bevorzugt die verbleibende Teilmenge des als Extraktionsmittel eingesetzten mindestens einen Dicarbonsäuredinitrils und das aus der wässrigen Phase extrahierte mindestens eine aliphatische, araliphatische oder cycloaliphatische Diamin enthält, einer Hydrierungsreaktion unterzogen, um das mindestens eine Dicarbonsäuredinitril zumindest teilweise in das entsprechende Diamin zu überführen. Bevorzugt werden in der Hydrierungsreaktion ≥ 80%, besonders bevorzugt ≥ 95% und ganz besonders bevorzugt ≥ 97% des mindestens einen Dicarbonsäuredinitrils zum entsprechenden Diamin umgesetzt. Bevorzugt erfolgt die Hydrierungsreaktion dabei an einem Katalysator auf Basis von Raney-Nickel, da diese Katalysatoren bekanntermaßen höhere Gehalte an Amin und Wasser, welches in der Regel ebenfalls in der organischen Phase aus der Extraktion enthalten ist, sehr gut tolerieren. Das extrahierte mindestens eine aliphatische, araliphatische oder cycloaliphatische Diamin und das aus der Hydrierung des mindestens einen Dicarbonsäuredinitrils entstandene zweite Diamin werden schließlich einer weiteren Reinigung unterzogen.

In einer besonders bevorzugten Ausführungsform wird das mindestens eine Dicarbonsäuredinitril so gewählt, dass bei der Hydrierung zumindest ein Teil des Dicarbonsäuredinitrils in das gleiche Amin umgesetzt wird, das aus der wässrigen Phase extrahiert wurde. Es wird also beispielsweise Adiponitril als Extraktionsmittel verwendet, wenn die wässrige Phase 1,6-Hexandiamin enthält, Glutaronitril, wenn sie 1,5-Pentandiamin enthält oder 2-Methylglutaronitril bzw. 2-Methylenglutaronitril, wenn sie 1,5-Diamino-2-methylpentan enthält. Auf diese Weise wird erreicht, dass in der Hydrierung nur ein Diamin als Hauptprodukt anfällt, so dass sich eine aufwändige Auftrennung verschiedener Diamine während der Reinigung der Produkte erübrigt.

Die Reinigung der Produkte aus der Hydrierung des mindestens einen Dicarbonsäuredinitrils in Gegenwart des mindestens einen aliphatischen Diamins erfolgt nach dem Fachmann an sich bekannten Verfahren. In der Regel erfolgt die Reinigung destillativ in einer oder mehreren Destillationskolonnen. Besonders vorteilhaft ist hier, dass das zuvor extrahierte mindestens eine aliphatische, araliphatische oder cycloaliphatische Diamin während der Hydrierung des Dicarbonsäuredinitrils ohne weiteres Zutun ebenfalls den Reduktionsbedingungen der Hydrierung unterzogen wird. Auf diese Weise können eventuell enthaltene polarographisch reduzierbare Verbindungen zumindest teilweise abgebaut und in der anschließenden Reinigung abgetrennt werden, was sich positiv auf die Produktqualität des Diamins und der daraus hergestellten Polymere auswirkt und Verfärbungen der Polymere entgegenwirkt. Ein solcher Effekt ist beispielsweise in der EP3235804A1 für eine katalytische Hydrierung 1,5-Pentandiamin enthaltend 2,3,4,5-Tetrahydropyridin beschrieben, wobei das 2,3,4,5-Tetrahydropyridin in Piperidin umgewandelt und dieses gegebenenfalls in einer anschließenden Destillation entfernt wird [Absätze 39-42].

In einer weiteren bevorzugten Ausführungsform der Erfindung wird das in der Hydrierung gewonnene Diamin, gegebenenfalls nach Durchlaufen eines oder mehrerer Zwischenschritte, mit Phosgen unter Bildung eines Diisocyanats umgesetzt. Optionale Zwischenschritte sind beispielsweise Reinigungsschritte, Trocknungsschritte oder Zwischenlagerungen. Sofern es sich um eine Mischung aus verschiedenen Diaminen handelt, das Dicarbonsäuredinitril also so gewählt war, dass das Produkt von dessen Hydrierung nicht dem zu extrahierenden mindestens einen aliphatischen, araliphatischen oder cycloaliphatischen Amin entspricht, oder schlicht eine Mischung aus verschiedenen Dicarbonsäuredinitrilien und/oder verschiedenen Diaminen eingesetzt wurde, ist es zwar möglich, diese Mischung von Diaminen mit Phosgen zum Diisocyanat umzusetzen, es ist jedoch bevorzugt, die Mischung vor der Phosgenierung beispielsweise in einer Destillation zu trennen und dann einzelne Diamine mit Phosgen umzusetzen.

Die Phosgenierung des Diamins kann beispielsweise in der Gasphase erfolgen. Dazu wird das Diamin bevorzugt verdampft und auf eine Temperatur innerhalb des Temperaturbereichs von 200 bis 600 °C erhitzt. Optional erfolgen die Verdampfung und auch der Einsatz der bei der Verdampfung erzeugten Diamin-Dämpfe in Gegenwart eines Inertgases und/oder von Dämpfen eines inerten Lösungsmittels. Bevorzugtes Inertgas ist Stickstoff. Geeignete inerte Lösungsmittel sind beispielsweise Chlorbenzol, o-Dichlorbenzol, Xylol, Chlornaphthalin oder deren Gemische.

Das bei der Phosgenierung verwendete Phosgen wird, bezogen auf die Aminogruppen, bevorzugt im Überschuss eingesetzt. Im Allgemeinen genügt eine Phosgenmenge, die 150 bis 350% der Theorie bezüglich der ablaufenden Phosgenierungsreaktion entspricht. Der Phosgenstrom wird vor der Reaktion bevorzugt auf eine Temperatur innerhalb des Bereichs von 200 bis 600 °C erhitzt.

Zur Durchführung der Phosgenierung werden der vorerhitzte, diaminhaltige Strom und der ebenfalls vorerhitzte Phosgenstrom bevorzugt kontinuierlich in einen zylindrischen Reaktionsraum geleitet und dort miteinander vermischt. Geeignete zylindrische Reaktionsräume sind beispielsweise Rohrreaktoren, welche im Allgemeinen aus Stahl, Glas, legiertem oder emailliertem Stahl bestehen. Sie weisen im Allgemeinen eine Länge auf, die ausreichend ist, um unter den Verfahrensbedingungen eine vollständige Umsetzung des Amins mit dem Phosgen zu ermöglichen. Des Weiteren sind die Abmessungen des Reaktionsraums bevorzugt so gewählt, dass im Reaktionsraum eine turbulente Strömung mit einer Reynoldszahl von mindestens 2500 vorherrscht. Dies ist im Allgemeinen dann gewährleistet, wenn die Strömungsgeschwindigkeit mehr als 90 m/s beträgt. Eine solche Strömungsgeschwindigkeit kann durch Einstellen eines entsprechenden Differenzdrucks zwischen den Produktleitungen zum Reaktionsraum und dem Ausgang aus dem Reaktionsraum sichergestellt werden. Im Allgemeinen liegt der Druck in den Zuleitungen bei 200 bis 300 mbar(ü) und am Ausgang aus dem Reaktionsraum bei 150 bis 200 mbar(ü).

Nach der erfolgten Phosgenierungsreaktion im Reaktionsraum wird das den Reaktionsraum kontinuierlich verlassende Gemisch bevorzugt von dem gebildeten Diisocyanat befreit. Dies kann beispielsweise durch selektive Kondensation in einem inerten Lösungsmittel wie beispielswiese Chlorbenzol oder Dichlorbenzol erfolgen. Sofern der diaminhaltige Strom bereits ein inertes Lösungsmittel enthielt, ist es bevorzugt, hier das gleiche Lösungsmittel zu verwenden. Die Temperatur des Lösungsmittels wird dabei bevorzugt so gewählt, dass sie einerseits oberhalb der Zersetzungstemperatur des dem Diisocyanat entsprechenden Carbamidsäurechlorids liegt und anderseits das Diisocyanat kondensiert bzw. sich in dem Lösungsmittel löst, während Phosgen, Chlorwasserstoff und gegebenenfalls Inertgas die Kondensationsstufe gasförmig durchlaufen. Besonders geeignet sind Lösungsmitteltemperaturen im Bereich von 120 bis 200 °C. Die erhaltene Diisocyanat-Rohlösung wird anschließend in einer mehrstufigen Destillation zum reinen Diisocyanat aufbereitet.

Das die Kondensationsstufe zur Gewinnung des mindestens einen Diisocyanates durchlaufende Gasgemisch wird anschließend bevorzugt in an sich bekannter Weise von überschüssigem Phosgen befreit. Dies kann mittels einer Kühlfalle, Absorption in einem bei einer Temperatur von -10 °C bis 8 °C gehaltenen inerten Lösungsmittel (z.B. Chlorbenzol (MCB), oder Dichlorbenzol (ODB) oder durch Adsorption und Hydrolyse an Aktivkohle erfolgen. Das die Phosgen-Rückgewinnungsstufe durchlaufende Chlorwasserstoffgas kann bevorzugt in an sich bekannter Weise zu Chlor oxidiert und dann beispielsweise wieder zu Phosgen umgesetzt werden.

In einer weiteren Ausführungsform erfolgt die Phosgenierung des Diamins in der flüssigen Phase. Die Reaktion kann dann auf verschiedene Weise ausgeführt werden. Entweder wird das Diamin direkt mit Phosgen in einem inerten flüssigen Medium umgesetzt (Basenphosgenierung) oder das Diamin wird zunächst durch Umsetzung mit Chlorwasserstoffgas oder Kohlendioxid in einem inerten flüssigen Medium das entsprechende Salz überführt und dann mit Phosgen umgesetzt (Hydrochlorid- bzw. Carbaminatphosgenierung). Als flüssiges Medium eigenen sich für alle Phosgenierungen insbesondere Chlorbenzol und/oder Dichlorbenzol.

Bei der Basenphosgenierung wird die Reaktion zweistufig in dem inerten flüssigen Medium durchgeführt. Im ersten Stadium, der Kalt-Phosgenierung, wird die Temperatur der Reaktionsmischung bevorzugt in einem Bereich zwischen 0 und 100 °C gehalten. Es entsteht eine Suspension, die Carbaminsäurechlorid, Aminhydrochlorid und geringe Mengen an freiem Diisocyanat enthält. Bevorzugt wird dabei eine Lösung von Phosgen in einem inerten Lösungsmittel vorgelegt und dann eine Lösung oder Suspension des Amins im gleichen Lösungsmittel sowie gegebenenfalls weiteres Phosgen zugegeben. Auf diese Weise wird die Konzentration von freiem Amin gering gehalten und somit die unerwünschte Bildung von Harnstoffen unterdrückt.

Im zweiten Stadium, der Heiß-Phosgenierung, wird die Temperatur erhöht und liegt vorzugsweise in einem Bereich von 120 bis 200 °C. Sie wird in diesem Bereich gehalten, während so lange weiteres Phosgen zugeführt wird, bis die Umsetzung zum Diisocyanat beendet ist, also die HCl-Entwicklung zum Erliegen kommt. Phosgen wird dabei zweckmäßig im Überschuss verwendet. Bei Bedarf kann die Reaktion sowohl in der Kalt- als auch in der Heißphosgenierung unter Einleitung eines inerten Gases durchgeführt werden.

Bei der Amin-Hydrochlorid- oder Carbaminat-Phosgenierung wird das Amin bevorzugt zunächst mit Chlorwasserstoffgas oder Kohlendioxid in einem inerten flüssigen Medium zur Erzeugung des entsprechenden Salzes umgesetzt. Die Reaktionstemperatur liegt während dieser Salzbildung vorzugsweise in einem Bereich von 0 bis 80 °C. Es schließt sich der Phosgenierschritt als zweiter Schritt an, der im Wesentlichen der Heiß-Phosgenierung aus der oben beschriebenen Basenphosgenierung ähnelt. Es wird also auch hier die Temperatur bevorzugt im Bereich von 120 bis 200 °C gehalten während Phosgen und optional ein Inertgas in die Reaktionsmischung eingeleitet werden. Die Einleitung erfolgt so lange, bis die Umsetzung zum Diisocyanat beendet ist. Auch hier wird Phosgen vorzugsweise im Überschuss eingesetzt, um die Reaktion zu beschleunigen.

Sowohl bei der Basenphosgenierung als auch bei der Amin-Hydrochlorid- bzw. der Carbaminat-Phosgenierung wird nach Beendigung der Reaktion das verbliebene Phosgen und Chlorwasserstoffgas bevorzugt mit einem Inertgas, vorzugsweise mit Stickstoff, ausgeblasen. Bei Bedarf kann eine Filtration erfolgen, um gegebenenfalls vorhandene Feststoffe wie unreagierte Amin-Hydrochloride zu entfernen. Das gebildete Diisocyanat wird anschließend in einer mehrstufigen Destillation zum reinen Diisocyanat aufbereitet.

In einer weiteren, bevorzugten Ausführungsform wird das Hydrierungsprodukt mit oder ohne Durchlaufen eines oder mehrerer Zwischenschritte mit einer Dicarbonsäure und/oder einem Dicarbonsäurederivat zu einem Polyamid umgesetzt. Bei dieser Ausführungsform wird das Diamin vorzugsweise, gegebenenfalls nach Durchlaufen eines oder mehrerer Zwischenschritte, in wässriger Lösung zusammen mit einer Dicarbonsäure erhitzt, und so in einer Polykondensationsreaktion zum Polyamid umgesetzt. Optionale Zwischenschritte sind beispielsweise Reinigungsschritte oder Zwischenlagerungen. Sofern es sich um eine Mischung aus verschiedenen Diaminen handelt, das Dicarbonsäuredinitril also so gewählt war, dass das Produkt von dessen Hydrierung nicht dem zu extrahierenden mindestens einen aliphatischen, araliphatischen oder cycloaliphatischen Amin entspricht, oder schlicht eine Mischung aus verschiedenen Dicarbonsäuredinitrilien und/oder verschiedenen Diaminen eingesetzt wurde, so kann diese Mischung wahlweise vor der Polykondensation in die einzelnen Diamine aufgetrennt werden oder als solche unter Bildung von Copolymeren eingesetzt werden.

In einer zum obigen Absatz alternativ bevorzugten Ausführungsform wird das Hydrierungsprodukt mit oder ohne Durchlaufen eines oder mehrerer Zwischenschritte mit wenigstens einem Epoxidharz umgesetzt. Dabei werden epoxidgruppenhaltige Verbindungen mit dem in der Hydrierung erhaltenen Diamin zu duroplastischen Kunststoffen umgesetzt. Die Umsetzung erfolgt besonders bevorzugt als Kalthärtung bei Raumtemperatur.

Ein weiterer Gegenstand der Erfindung ist die Verwendung mindestens eines Dicarbonsäuredinitrils zur Extraktion mindestens eines aliphatischen, araliphatischen oder cycloaliphatischen Diamins aus einer Mischung enthaltend mindestens 40 Gew.-% Wasser und 0,2 bis 40 Gew.-% des mindestens einen aliphatischen, araliphatischen oder cycloaliphatischen Diamins unter Erhalt einer wässrigen Phase enthaltend Wasser sowie eine Teilmenge des mindestens einen Dicarbonsäuredinitrils und einer organischen Phase enthaltend eine weitere, bevorzugt die verbleibende Teilmenge des mindestens Dicarbonsäuredinitrils sowie das mindestens eine aliphatische, araliphatische oder cycloaliphatische Diamin.

Eine bevorzugte Ausführungsform des Verfahrens ist die Verwendung von 2-Methylglutaronitril und/oder 2-Methylenglutaronitril zur Extraktion von 1,5-Diamino-2-methylpentan aus einer wässrigen Phase enthaltend 1,5-Diamino-2-methylpentan.

Eine weitere bevorzugte Ausführungsform des Verfahrens ist die Verwendung von Glutaronitril zur Extraktion von 1,5-Pentandiamin aus einer wässrigen Phase enthaltend 1,5-Pentandiamin.

Eine weitere bevorzugte Ausführungsform des Verfahrens ist die Verwendung von Succinonitril zur Extraktion von 1,4-Butandiamin aus einer wässrigen Phase enthaltend 1,4-Butandiamin.

Eine weitere bevorzugte Ausführungsform des Verfahrens ist die Verwendung von Adiponitril zur Extraktion von 1,6-Hexandiamin aus einer wässrigen Phase enthaltend 1,6-Hexandiamin.

Ein weiterer Gegenstand der Erfindung ist darüber hinaus die Verwendung der organischen Phase enthaltend eine weitere Teilmenge, bevorzugt die verbleibende Teilmenge, des mindestens einen Dicarbonsäuredinitrils und das mindestens eine aliphatische, araliphatische oder cycloaliphatische Diamin als Einsatzstoff in einer Hydrierung zur Herstellung eines aliphatischen Diamins oder einer Mischung von aliphatischen Diaminen.

Ein besonders vorteilhafter erfindungsgemäßer Prozess soll im Folgenden beispielhaft erläutert werden:
Auf biochemischem Wege wird 1,5-Pentandiamin durch Decarboxylierung von L-Lysin hergestellt. Aus der Fermentationsbrühe werden zunächst zelluläre Bestandteile durch Filtration entfernt, so dass eine wässrige Mischung enthaltend ca. 20 Gew.-% 1,5-Pentandiamin erhalten wird. Diese Mischung wird durch Zugabe von Natriumhydroxid auf pH 11 eingestellt und bei 25 °C im Gegenstrom in einer mehrstufigen Extraktionskolonne mit Glutaronitril extrahiert. Dabei fällt am einen Ende der Extraktionskolonne ein wässriger Strom enthaltend ca. 10 Gew.-% Glutaronitril und 0,08 Gew.-% Pentandiamin an. Am anderen Ende der Extraktionskolonne wird eine organische Phase enthaltend Glutaronitril, 1,5-Pentandiamin und ca. 10 Gew.-% Wasser.

Der wässrige Strom aus der Extraktion wird einem Verfahren zur Herstellung von Glutaronitril durch Reaktion von 1,3-Dichlorpropan mit Kaliumcyanid zur Verfügung gestellt. Dieses Verfahren umfasst einen Reinigungsschritt, in dem die Reaktionsmischung mit Wasser und Chloroform extrahiert wird und der wässrige Storm aus der Extraktion ersetzt hier einen Teil des Wassers. Dabei wird auch das Glutaronitril aus dem wässrigen Strom zurückgewonnen und dann zusammen mit dem Glutaronitril aus der Umsetzung des 1,3-Dichlorpropans weiter aufgearbeitet.

Die organische Phase enthaltend Glutaronitril, ca. 25 Gew.-% 1,5-Pentandiamin und ca. 10 Gew.-% Wasser wird dann gemäß der Lehre von DE69413560T2 einer katalytischen Hydrierung unter Verwendung eines Titan-dotierten Raney-Nickel-Katalysators bei 80 °C und 20 bar Wasserstoffdruck hydriert. Unter diesen Bedingungen wird das Glutaronitril vollständig zu 1,5-Pentandiamin umgesetzt und auch Spuren von polarographisch reduzierbaren Verbindungen werden reduziert, also beispielsweise 2,3,4,5-Tetrahydropyridin zum Piperidin. Das Produkt der Hydrierungsreaktion wird in einer Vakuumdestillation gereinigt und man erhält 1,5-Pentandiamin in einer für die Herstellung von 1,5-Pentandiisocyanat durch Phosgenierung geeigneten Qualität. Dieses Produkt wird einer Gasphasenphosgenierung wie in EP3194362 beschrieben umgesetzt und man erhält nach destillativer Reinigung 1,5-Pentandiisocyanat.

## Patentansprüche

1. Verfahren zur Gewinnung mindestens eines aliphatischen, araliphatischen oder cycloaliphatischen Diamins aus einer Mischung enthaltend mindestens 35 Gew.-% Wasser und 0,2 bis 60 Gew.-% des mindestens einen aliphatischen, araliphatischen oder cycloaliphatischen Diamins durch Extraktion des mindestens einen aliphatischen, araliphatischen oder cycloaliphatischen Diamins mit mindestens einem Dicarbonsäuredinitril unter Erhalt einer wässrigen Phase enthaltend Wasser sowie eine Teilmenge des mindestens einen Dicarbonsäuredinitrils und einer organischen Phase enthaltend eine weitere Teilmenge des mindestens einen Dicarbonsäuredinitrils sowie das mindestens eine aliphatische, araliphatische oder cycloaliphatische Diamin.

2. Verfahren nach Anspruch 1, **dadurch gekennzeichnet, dass** das mindestens eine Dicarbonsäuredinitril ein Dicarbonsäuredinitril gemäß der Formel (I) ist,
NC-R-CN (I),
wobei R für einen Alkylrest mit 1 bis 12 Kohlenstoffatomen oder einen Alkenylrest mit 4 bis 8 Kohlenstoffatomen oder einen Arylrest mit 6 Kohlenstoffatomen steht.

3. Verfahren nach einem der Ansprüche 1 oder 2, **dadurch gekennzeichnet, dass** das mindestens eine Dicarbonsäuredinitril ausgewählt ist aus der Gruppe bestehend aus Succinonitril, Glutaronitril, Adiponitril, Sebaconitril, Metyhlglutaronitril, Ethylsuccinonitril und Methylenglutaronitril.

4. Verfahren nach einem der Ansprüche 1 bis 3, **dadurch gekennzeichnet, dass** die Mischung, einen Gehalt des mindestens einen aliphatischen, araliphatischen oder cycloaliphatischen Diamins von ≤ 40 Gew.-% und ≥ 0,5 Gew.-%, bezogen auf die Gesamtmasse der Mischung, aufweist.

5. Verfahren nach einem der Ansprüche 1 bis 4, **dadurch gekennzeichnet, dass** das mindestens eine aliphatische, araliphatische oder cycloaliphatische Diamin ausgewählt ist aus der Gruppe bestehend aus 1,4-Butandiamin, Neopentandiamin, 1,5-Pentandiamin, 1,5-Diamino-2-methylpentan, 1,5-Diamino-2-butyl-2-ethylpentan, 1,6-Hexandiamin, 2,5-Diamino-2,5-dimethylhexan, 1-Amino-3,3,5-trimethyl-5-aminomethyl-cyclohexan, 1,4-Diaminocyclohexan, 2,4-Diamino-1-methylcyclohexan, 2,6-Diamino-1-methylcyclohexan, m-Hexahydroxylylendiamin, 1,6-Diamino-2,2,4-trimehtylhexan, 1,6-Diamino-2,4,4-trimethylhexan, 2,4'-Diaminodicyclohexylmethan, 4,4'-Diaminodicyclohexylmethan, Bis(aminomethyl)bicyclo[2.2.1]heptan, 1,8-Diaminooctan, 1,9-Diaminononan, 1,10-Diaminodecan, 1,11-Diaminoundecan, 1,12-Diaminododecan und m-Xylylendiamin.

6. Verfahren nach einem der Ansprüche 1 bis 5, **dadurch gekennzeichnet, dass** die Extraktion im alkalischen Milieu erfolgt.

7. Verfahren nach einem der Ansprüche 1 bis 6, **dadurch gekennzeichnet, dass** die organische Phase enthaltend die weitere, bevorzugt die verbliebene Teilmenge des mindestens einen Dicarbonsäuredinitrils und das extrahierte mindestens eine aliphatische, araliphatische oder cycloaliphatische Diamin und Wasser, gegebenenfalls nach Durchlaufen eines oder mehrerer Zwischenschritte, einer Hydrierungsreaktion unter Erhalt eines Hydrierungsproduktes, enthaltend das mindestens eine aliphatische, araliphatische oder cycloaliphatische Diamin unterzogen wird.

8. Verfahren nach Anspruch 7, **dadurch gekennzeichnet, dass** das mindestens eine Dicarbonsäuredinitril so gewählt ist, dass in der Hydrierung zumindest ein Teil des Dicarbonsäuredinitrils in das gleiche Diamin umgesetzt wird, das aus der wässrigen Phase extrahiert wurde.

9. Verfahren nach Anspruch 7 oder 8, **dadurch gekennzeichnet, dass** das Hydrierungsprodukt mit oder ohne Durchlaufen eines oder mehrerer Zwischenschritte mit Phosgen unter Bildung eines Diisocyanats umgesetzt wird.

10. Verfahren nach Anspruch 7 oder 8, **dadurch gekennzeichnet, dass** das Hydrierungsprodukt mit oder ohne Durchlaufen eines oder mehrerer Zwischenschritte mit einer Dicarbonsäure und/oder einem Dicarbonsäurederivat zu einem Polyamid umgesetzt wird.

11. Verfahren nach Anspruch 7 oder 8, **dadurch gekennzeichnet, dass** das Hydrierungsprodukt mit oder ohne Durchlaufen eines oder mehrerer Zwischenschritte mit wenigstens einem Epoxidharz umgesetzt wird.

12. Verwendung mindestens eines Dicarbonsäuredinitrils zur Extraktion mindestens eines aliphatischen, araliphatischen oder cylcoaliphatischen Amins aus einer Mischung enthaltend mindestens 35 Gew.-% Wasser und 0,2 zu 60 Gew.-% des mindestens einen aliphatischen, araliphatischen oder cycloaliphatischen Diamins unter Erhalt einer wässrigen Phase enthaltend Wasser sowie eine Teilmenge des mindestens einen Dicarbonsäuredinitrils und einer organischen Phase enthaltend eine weitere Teilmenge, bevorzugt die verbleibende Teilmenge, des mindestens einen Dicarbonsäuredinitrils sowie das mindestens eine aliphatische, araliphatische oder cylcoaliphatische Diamin.

13. Verwendung der organischen Phase enthaltend eine weitere Teilmenge, bevorzugt die verbleibende Teilmenge, des mindestens einen Dicarbonsäuredinitrils und das mindestens eine aliphatische, araliphatische oder cycloaliphatische Diamin als Einsatzstoff in einer Hydrierung zur Herstellung eines aliphatischen Diamins.

14. Organische Phase, erhältlich oder hergestellt nach in einem Verfahren gemäß einem der Ansprüche 1 bis 6, enthaltend eine weitere Teilmenge, bevorzugt die verbleibende Teilmenge, des mindestens einen Dicarbonsäuredinitrils, das mindestens eine aliphatische, araliphatische oder cycloaliphatische Diamin und Wasser.

15. Organische Phase, bestehend zu 1 bis 50 Gew.-% aus mindestens einem aliphatischen, araliphatischen oder cycloaliphatischen Diamin, zu 0,2 bis 25 Gew.-% aus Wasser und zu 0,01 bis 5 Gew.-% aus Verunreinigungen, wobei der zu 100 Gew.-% fehlende Teil der organischen Phase aus mindestens einem Dicarbonsäuredinitril besteht.
